# EUROPÄISCHE PATENTANMELDUNG

(11) **EP 0 826 390 A2**
(43) Veröffentlichungstag der Anmeldung: **04.03.1998**
(21) Anmeldenummer: 97810466.9
(22) Anmeldetag: 11.07.1997
(51) Int. Cl.: A61N 1/05, A61N 1/04, A61B 17/39

(54) **Verbindungselement für ein äusseres Endstück einer chirurgischen Elektrode**

(30) Priorität: 06.08.1996 EP 96810517
(71) Anmelder: Sulzer Osypka GmbH, 79639 Grenzach-Wyhlen (DE)
(72) Erfinder: Osypka, Peter, Dr., 79639 Grenzach-Wyhlen (DE)
(74) Vertreter: Heubeck, Bernhard

(57) **Zusammenfassung**

Das Verbindungselement (1) für ein äusseres Endstück (42) einer chirurgischen Elektrode (4) ist zum Anbringen an der Körperoberfläche eines Herzpatienten (70) vorgesehen. Es umfasst Mittel, mit denen eine mechanische und elektrische Verbindung zwischen dem äusseren Endstück und einer Anschlussbuchse (20) herstellbar ist. Mit Vorteil werden zum zeitweiligen Überwachen und/oder Stimulieren der Herztätigkeit eine bzw. mehreren unipolare Elektroden (4) verwendet. Der oder jeder unipolaren Elektrode ist eine auf der Körperoberfläche angeordnete indifferente Elektrode (11) zugeordnet, die vorzugsweise auf einer Grundplatte des Verbindungselements angeordnet ist.

## Beschreibung

Die Erfindung betrifft ein Verbindungselement für ein äusseres Endstück einer chirurgischen Elektrode gemäss Oberbegriff von Anspruch 1 sowie Verwendungen derartiger Verbindungselemente.

Eine chirurgischen Elektrode, die auch Herzdraht genannt wird, ist aus der europäischen Patentschrift EP 0 159 540 bekannt. Der Herzdraht wird nach Herzoperationen zur zeitweiligen Überwachung und Stimulierung der Herztätigkeit verwendet. Er bildet eine isolierte elektrische Verbindung zwischen zwei unisolierten Enden. Das eine Ende ist im Herzmuskel verankert. Das andere Ende - das äussere Endstück - befindet sich ausserhalb des Brustkorbs, wo es an einen äusseren Herzschrittmacher oder EKG-Monitor anschliessbar ist.

Bei der Verwendung von unipolaren Elektroden ist jeweils nur ein Herzdraht nötig. In der Regel werden zwei unipolare Elektroden verwendet, die eine für den Vorhofbereich des Herzens und die andere für den Ventrikelbereich. Jeder unipolaren Elektrode ist eine indifferente Elektrode zugeordnet, die an der Körperoberfläche des Patienten angebracht wird. Bei Verwendung langer Elektroden werden - bis zum Anschluss an die genannten Geräte - die äusseren Teile der Drähte aufgewickelt und ohne besondere Massnahmen an dem Bauch des Patienten mit Heftpflaster befestigt. Falls die Elektroden kurz sind (60 cm), müssen für die externe Stimulation Verlängerungskabel angebracht werden, da sich der Stimulator und der EKG-Monitor nicht am Patienten sondern in einem gewissen Abstand in Bettnähe befindet. Dabei entsteht oft ein Kabelgewirr, das zu einer Verwechslung der Kabel führen kann.

Es ist daher Aufgabe der Erfindung, Mittel zu schaffen, die eine zuverlässigere Behandlung von Herzpatienten bei der Verwendung von chirurgischen Elektroden ermöglichen. Diese Aufgabe wird mit dem in Anspruch 1 definierten Verbindungselement gelöst. Mit erfindungsgemässen Verbindungselementen kann jede Elektrode mit jeweils einer Anschlussbuchse verbunden werden. Diese Verbindungselemente erlauben eine übersichtliche Anordnung der Anschlussstellen, eine schnelle Verbindung mit Verlängerungskabeln durch Zusammenstecken und damit einen sicheren sowie schnellen Anschluss an den Stimulator bzw. EKG-Monitor. Es lassen sich relativ kurze Herzdrähte (Länge rund 60 cm) verwenden, wodurch eine gute Übersichtlichkeit ermöglicht wird.

Das erfindungsgemässe Verbindungselement für ein äusseres Endstück einer chirurgischen Elektrode ist zum Anbringen an der Körperoberfläche eines Herzpatienten vorgesehen. Es umfasst Mittel, mit denen eine mechanische und elektrische Verbindung zwischen dem äusseren Endstück und einer Anschlussbuchse ist. Mit Vorteil werden zum zeitweiligen Überwachen und/oder Stimulieren der Herztätigkeit eine bzw. mehreren unipolare Elektroden verwendet. Der oder jeder unipolaren Elektrode ist eine auf der Körperoberfläche angeordnete indifferente Elektrode zugeordnet, die vorzugsweise auf einer Grundplatte des Verbindungselements angeordnet ist.

Die Ansprüche 2 bis 10 beziehen sich auf besondere und vorteilhafte Verbindungselemente. Gegenstand der Ansprüche 11 bis 14 ist jeweils eine Verwendung von erfindungsgemässen Verbindungselementen.

Nachfolgend wird die Erfindung anhand der Zeichnungen erläutert. Es zeigen:
- Fig. 1: ein erfindungsgemässes Verbindungselement mit zwei Anschlussbuchsen - ein sogenanntes Terminal,
- Fig. 2: einen Schnitt durch das Terminal der Fig.1 nach der Linie II-II,
- Fig. 3: einen zweiten Schnitt durch das Terminal nach der Linie III-III,
- Fig. 4: einen unipolarer Herzdraht (chirurgische Elektrode),
- Fig. 5: eine Draufsicht auf ein Terminal mit angeschlossenem Herzdraht,
- Fig. 6: eine Darstellung, wie die zwei Terminals am Herzpatienten anzuordnen sind,
- Fig. 7: eine Armmanschette, die als erfindungsgemässes Verbindungselement ausgebildet ist und

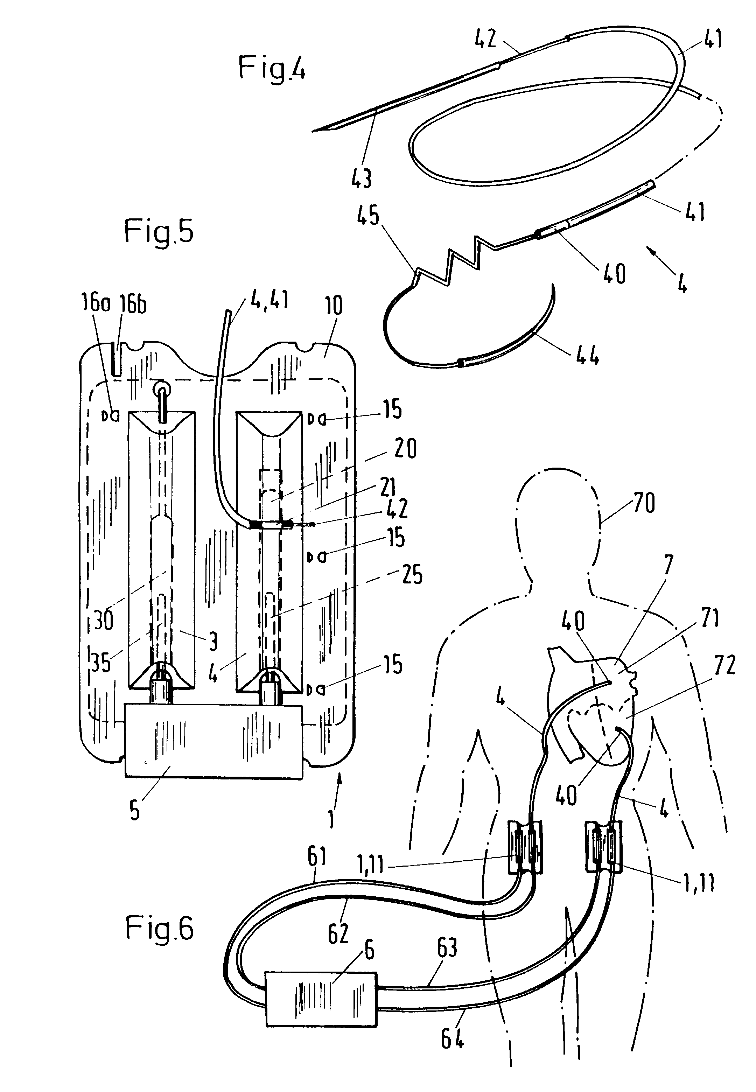

- Fig. 6: eine zweite Ausführungsform einer derartigen Armmanschette.

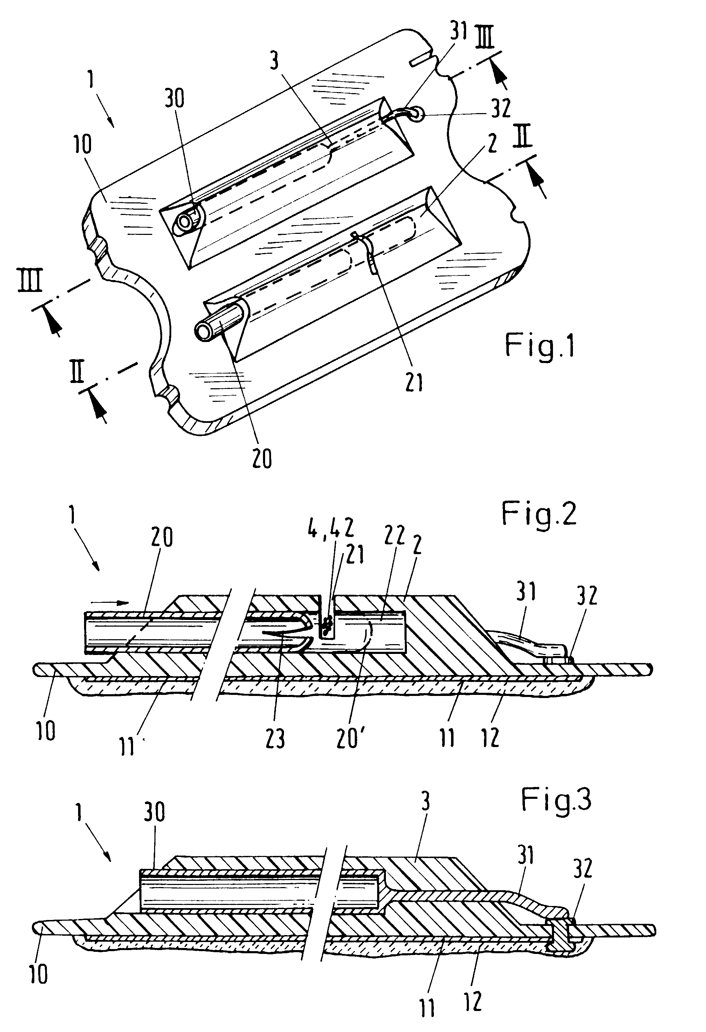

Das erfindungsgemässe Verbindungselement oder Terminal 1 der Fig.1 zeigt folgende Teile: eine Grundplatte 10; eine Verbindungsstelle 2 für einen Herzdraht 4 (siehe Fig.4) und eine Anschlussbuchse 20; und eine Verbindungsstelle 3 für eine Anschlussbuchse 30 und eine indifferente Elektrode (11, siehe Fig.2), die auf der Unterseite der Grundplatte 10 angeordnet ist und die mit der Buchse 30 über einen Draht 31 und ein Element 32 verbunden ist. Die Verbindungsstelle 2 weist eine schlitzförmige Einlegeöffnung 21 für ein äusseres Endstück 42 des Herzdrahts 4 auf.

Auf der Unterseite der Grundplatte 10 können Stellen vorgesehen sein, die mit einem hautfreundlichen, gegebenfalls elektrisch leitfähigen Klebstoff beschichtet sind und die ein einfaches Befestigen des Terminals 1 am Patienten ermöglichen.

Der Längsschnitt durch die Verbindungsstelle 2 in Fig.2 zeigt die bewegliche Anschlussbuchse 20, die in einem Kanal 22 durch dessen Wände geführt verschiebbar ist. Die Einlegeöffnung 21 für das äussere Endstück 42 des Herzdrahts 4 (eine Litze) ist in einem Bereich des Kanals 22 angeordnet, in den die Buchse 20 einschiebbar ist. Eine mechanische und elektrische Verbindung zwischen dem äusseren Endstück 42 und der Anschlussbuchse 20 ergibt sich durch Einschieben der Buchse 20 und Verklemmen des Endstütcks 42 in einem keilförmigen Schlitz 23 der Buchse 20. Die Position der Buchse 20 nach dem Einschieben ist durch die strichpunktierte Linie 20' angedeutet. Die Verbindung zwischen dem Herzdraht und der Buchse 20 kann auch anders, beispielsweise durch Verklemmen des Endstücks 42 zwischen der Buchse 20 und der Innenwand des Kanals 22 hergestellt werden.

Fig.3 zeigt den Längsschnitt durch die Verbindungsstelle 3, die die Anschlussbuchse 30 mit der indifferenten Elektrode 11 verbindet (Draht 31, Element 32). Die Elektrode 11 ist von einem elektrisch leitenden Gel 12 bedeckt, das für einen einwandfreien Kontakt mit der Körperoberfläche des Patienten vorgesehen ist und das gleichzeitig einen hautfreundlichen Klebstoff darstellt.

Die chirurgische Elektrode 4 oder der Herzdraht 4, der in Fig.4 gezeigt ist, ist eine bekannte unipolare Elektrode und weist folgende Teile auf: eine isolierte Litze 41 mit einer Länge von beispielsweise 60 cm; ein unisoliertes Litzenstück, das das äussere Endstück 42 bildet; eine differente Elektrode 40 in Form einer Hülse aus Platin, mit der ein elektrischer Kontakt zum Herzmuskel herstellbar ist; und ein zick-zack-förmiger Kunststofffaden, mit dem der Herzdraht 4 im Herzmuskel verankerbar ist. An den beiden Enden des Herzdrahts 4 befinden sich eine Brustnadel 43 bzw. eine Herznadel 44, die Operationshilfen sind und die nach der zweckgemässen Verwendung jeweils abgeschnitten werden.

Das Terminal 1 der Fig.5 ist mit dem Herzdraht 4 verbunden. Ein tragbares Gerät 5 ist direkt an Buchsen 20 und 30 angeschlossen. Dieses Gerät 5 ist ein EKG-Verstärker und ferngesteuerter Stimulator, dessen Steckstifte 25 und 35 anstelle von Verlängerungskabeln in die Buchsen 20 bzw. 30 eingeschoben sind. Der Stimulator kann von aussen programmiert werden, beispielsweise über HF-Telemetrie-Signale oder mittels einer Infrarot-Fernbedienung.

In Fig.5 ist gestrichelt der Rand der indifferenten Elektrode 11 angegeben. Ferner sind Aufnahmeelemente 15 und 16a sowie 16b gezeigt, die zum Einsatz kommen, wenn das Terminal 1 als Teil einer Verpackung für die chirurgische Elektrode 4 verwendet wird, nämlich als Aufwickelkörper für die Elektrode 4. Die Elemente 15 sind für die Aufnahme der Brustnadel 43, die Elemente 16a, 16b für die Aufnahme der Herznadel 44 vorgesehen. Die Grundplatte 10 bildet zusammen mit den Anschlussstellen 2 und 3 einen geeignet geformten Aufwickelkörper für den Draht 41.

Fig.6 stellt die Verwendung zweier erfindungsgemässen Verbindungselemente 1 gemäss der oben beschriebenen Ausführungsform dar. Der Patient mit dem Herz 7 ist durch die strichpunktierte Umrisslinie 70 angedeutet. Diese Verwendung betrifft das zeitweilige Überwachen und/oder Stimulieren der Herztätigkeit mit unipolaren Elektroden 4 und mit auf der Körperoberfläche angeordneten indifferenten Elektroden 11. Dabei ist die eine Elektrode 4 für die Behandlung des Vorhofbereichs 71 des Herzens 7 und die andere für die Behandlung des Ventrikelbereichs 72 eingesetzt. Die Terminals 1 sind über Kabel 61, 62, 63 und 64 mit einem Stimulator 6 verbunden. Die Kabel 61 und 63 stellen jeweils die Verbindungen zu den indifferenten Elektroden 11 her, die Kabel 62 und 64 zu den differenten Elektroden 40. Werden tragbare Geräte 5 gemäss der Fig.5 verwendet, so wird eine Synchronisation zwischen den Geräten 5 hergestellt, beispielsweise mittels Telemetriesignalen.

Mit Vorteil wird das Terminal als Kunststoffspritzteil aus Polyethylen gefertigt. Die Buchsen können aus Messing mit anschliessender Vergoldung hergestellt werden werden.

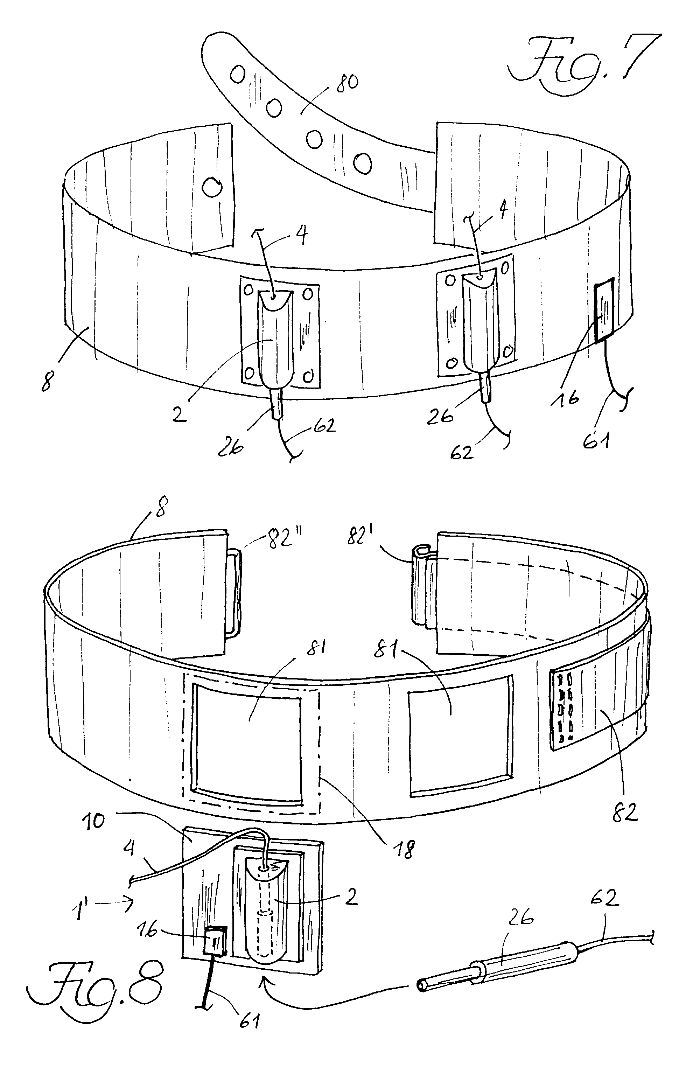

Eine weitere vorteilhafte Ausführungsform des erfindungsgemässen Verbindungsteils 1 ist in Fig.7 dargestellt: eine Armmanschette 8 mit zwei Anschlussbuchsen 2, die über die Drähte 4 mit dem Herz verbindbar sind, und mit Steckern 26 sowie Drähten 62 (vgl. Fig.8), mit welchen eine Verbindung zu einem nicht dargestellten Stimulator herstellbar ist. Mit einem Verschlussriemen 80 lässt sich die Manschette 8 am Oberarm des Patienten, vorzugsweise am linken Oberarm, anbringen. Das Material der Manschette 8 ist elektrisch leitfähig, so dass diese - auf der Haut des Patienten aufliegend - als eine gemeinsame indifferente Elektrode verwendbar ist. Ein entsprechender elektrischer Kontakt 16 sowie ein Draht 61 bilden eine Verbindung zwischen der indifferenten Elektrode und dem Stimulator. Der elektrische Kontakt 16 kann fest oder als Stecker ausgebildet sein.

Eine weitere Armmanschette 8 ist in Fig.8 gezeigt. Sie besteht aus einem elektrisch isolierenden Material und weist Fenster 81 für Verbindungselemente 1' auf. Jedes Verbindungselement 1' umfasst eine als indifferente Elektrode vorgesehene, elektrisch leitende Grundplatte 10 - mit Vorteil aus einem leitfähigen Kunststoff (Gummi, Silikon) - und einen elektrischen Anschluss 16, 61 an den Stimulator. Die auf der Grundplatte 10 befestigte Anschlussbuchse 2 mit dem Verbindungsdraht 4 und dem Stecker 26 ist gleich wie jene der Manschette 8 in Fig.7 ausgebildet. Das Element 1' wird unter die Manschette 8 in das Fenster 81 geschoben, wo es einen festen Sitz einnimmt. Der Rand 18 des unter der Manschette 8 angeordneten Elements 1' ist strichpunktiert dargestellt. Die Manschette 8 wird in diesem Beispiel mittels Haken 82' und Öse 82" geschlossen, wobei der Haken 82' über ein elastisches Band 82 an der Manschette 8 befestigt ist.

## Patentansprüche

1. Verbindungselement (1) für ein äusseres Endstück (42) einer chirurgischen Elektrode (4), welches zum Anbringen an der Körperoberfläche eines Herzpatienten (70) vorgesehen ist und welches Mittel umfasst, mit denen eine mechanische und elektrische Verbindung zwischen dem äusseren Endstück und einer Anschlussbuchse (20) herstellbar ist.

2. Verbindungselement nach Anspruch 1, dadurch gekennzeichnet, dass die Anschlussbuchse (20) beweglich und in einem Kanal (22) durch dessen Wände geführt verschiebbar ist, und dass eine Einlegeöffnung (21) für das äussere Endstück (42) in dem Kanal angeordnet ist, welche in einem Bereich liegt, in den die Buchse einschiebbar ist.

3. Verbindungselement nach Anspruch 1 oder 2, dadurch gekennzeichnet, dass eine zweite Anschlussbuchse (30) mit einer indifferenten, an der Körperoberfläche des Patienten angeordneten Elektrode (11) verbunden ist.

4. Verbindungselement nach einem der Ansprüche 1 bis 3, dadurch gekennzeichnet, dass die oder jede Anschlussbuchse (20, 30) auf einer Grundplatte (10) angeordnet ist, die für das Aufbringen des Verbindungselements (1) auf der Körperoberfläche vorgesehen ist.

5. Verbindungselement nach einem der Ansprüche 1 bis 4, dadurch gekennzeichnet, dass hautfreundliche Haftmittel auf der Unterseite der Grundplatte (10) zum Fixieren auf der Körperoberfläche vorgesehen sind.

6. Verbindungselement nach Anspruch 3, dadurch gekennzeichnet, dass die indifferente Elektrode (11) Teil des Verbindungselements (1) ist.

7. Verbindungselement nach einem der Ansprüche 1 bis 6, dadurch gekennzeichnet, dass es als Aufwickelkörper für die Elektrode (4) ausgebildet ist.

8. Verbindungselement nach einem der Ansprüche 1 bis 3, dadurch gekennzeichnet, dass die oder jede Anschlussbuchse (20, 30) auf einer Armmanschette (8) angeordnet ist.

9. Verbindungselement nach Anspruch 8, dadurch gekennzeichnet, dass die Armmanschette (8) als indifferente Elektrode ausgebildet ist.

10. Verbindungselement nach Anspruch 9, dadurch gekennzeichnet, dass die Armmanschette (8) fensterartige Aussparungen aufweist, in die separierbare Elemente mit Anschlussbuchsen einsetzbar sind.

11. Verwendung eines Verbindungselements oder mehrerer Verbindungselemente gemäss einem der Ansprüche 1 bis 10 zum zeitweiligen Überwachen und/oder Stimulieren der Herztätigkeit mit einer bzw. mehreren unipolaren Elektroden (4), wobei der oder jeder unipolare Elektrode eine auf der Körperoberfläche angeordnete indifferente Elektrode (11) zugeordnet ist.

12. Verwendung von Verbindungselementen gemäss Anspruch 11, dadurch gekennzeichnet, dass ein tragbares, direkt an die oder jede Buchse (20, 30) anschliessbares Gerät (5) als EKG-Verstärker und Stimulator eingesetzt wird.

13. Verwendung von Verbindungselementen gemäss Anspruch 11 oder 12, dadurch gekennzeichnet, dass der Einsatz von zwei unipolaren Elektroden (4) - die eine für den Vorhofbereich (71) des Herzens (7) und die andere für den Ventrikelbereich (72) - vorgesehen sind.

14. Verwendung von Verbindungselementen gemäss den Ansprüchen 12 und 13, dadurch gekennzeichnet, dass jeder Elektroden (4) je ein Gerät (5) der genannten Art zugeordnet ist und dass mittels Telemetriesignalen eine Synchronisation zwischen den Geräten herstellbar ist.
